# EUROPEAN PATENT SPECIFICATION

(11) **EP 4 384 253 B1**
(45) Date of publication and mention of the grant of the patent: **20.11.2024**
(21) Application number: 22777031.0
(22) Date of filing: 03.08.2022
(51) Int. Cl.: A61M 25/10

(54) **OVERMOLDED BALLOON ATTACHMENT TO SHAFT**
ÜBERGOSSENE BALLONBEFESTIGUNG AN EINEM SCHAFT
FIXATION DE BALLONNET SURMOULÉ À UN ARBRE

(30) Priority: 13.08.2021 US 202163232890 P
(43) Date of publication of application: 19.06.2024
(73) Proprietor: CooperSurgical, Inc., Trumbull, CT 06611 (US)
(72) Inventor: DALY, Mark, D., Ellettsville, 47429 (US); CONKLIN, Stephen, Bloomington, 47403 (US)
(74) Representative: Fish & Richardson P.C.
(86) International application number: PCT/US2022/039249
(87) International publication number: WO 2023/018585

(56) References cited:
- JP-A- 2002 143 311
- US-A1- 2017 100 570
- US-A1- 2019 030 282
- US-A1- 2019 142 246

## Description

### Technical Field

This disclosure relates to attachment of a balloon to a shaft via overmolding.

### Background

In various systems, an inflatable balloon attached to shaft may be inserted into the body and inflated to control fluid flow. In some cases, the balloon may be inflated to pressures at which weak and/or defective balloons may burst or become detached from the shaft. In some cases, such a failure event may lead to harm to the individual undergoing the procedure and/or require repeat of various portions of the procedure. Accordingly, technologies that decrease the likelihood of failures and/or increase the operational tolerances of such inflatable balloon devices will drive demand for these devices.

US 2017/0100570 describes a cage that can be positioned around a medical balloon, such as an angioplasty balloon, to assist in a medical procedure. The cage can include a plurality of strips, each extending between a set of rings including first and second rings. As the balloon expands, the first and second rings move closer together and allow the strips to expand outward. The cage may have wedge dissectors on the strips.

US 2019/0142246 describes a visualization catheter that includes an elongate shaft, a cap portion, and an offset balloon coupled to the distal end of the shaft. The elongate shaft includes a proximal end, a distal end, and a central axis defined therebetween. The cap portion is coupled to the distal end of the shaft. The cap portion also includes a visualization element and defines an aperture. The offset balloon is coupled to the distal end of the shaft and encapsulates the cap portion. The offset balloon defines a center point offset relative to the central axis of the shaft.

US 2019/0030282 describes methods for predicting the risk of recurrence and/or metastasis in primary cutaneous squamous cell carcinoma.

JP 2002-143311 describes a balloon catheter wherein both ends of the balloon are fixed onto an outer surface near a point of the flexible shaft by the fixing means, the fixing strength of the basic end-side fixing part of the balloon is weaker than that of the point-side fixing part in fixing the both ends of the balloon.

### BRIEF DESCRIPTION OF THE DRAWINGS

Figure 1 shows a cross-sectional view of an example inflatable device.
Figure 2 shows an alternate solid view of the example inflatable device.
Figure 3 shows an example mold.
Figure 4 shows an example after-molding device.
Figure 5 shows an example method for fabricating an inflatable device.
Figure 6 shows a detail view of example shut-offs.
Figure 7 shows an example of a completed inflation device.

### DETAILED DESCRIPTION

In various contexts, an inflatable balloon may be attached to a shaft to form an inflatable device. The shaft may include a catheter and/or lumen. The inflatable device may be inserted into the body and inflated to control fluid flow surrounding the inflatable device. The balloon may be attached to the shaft, for example, using an adhesive and/or via overmolding.

The conventional wisdom is to attach the balloon without pre-defined shaping of the attachment medium, allowing flow and/or expansion of the attachment medium to an equilibrium state. Contrary to conventional wisdom, the devices and fabrication techniques disclosed herein are formed with and/or use a shut-off that constrains the flow and/or expansion of the attachment medium during overmolding, which may restrict the attachment surface area. Accordingly, the resultant overmold extends from the balloon to a shut-off ridge. The shut-off ridge may include a defined shape formed through the constrained shaping provided by the shut-off during overmolding. The overmold with the shut-off ridge does not undergo unconstrained expansion and does not include bubbles. Unexpectedly, the resultant overmold without bubbles has a higher burst strength to similarly sized attachments made with adhesives, which can form bubbles due to expansion. Further, the shaping of the shut-off ridges prevents flash on the ends of the overmold. In the medical context, flash can injure tissue during a medical procedure, which can lead to dangerous conditions such as thrombosis.

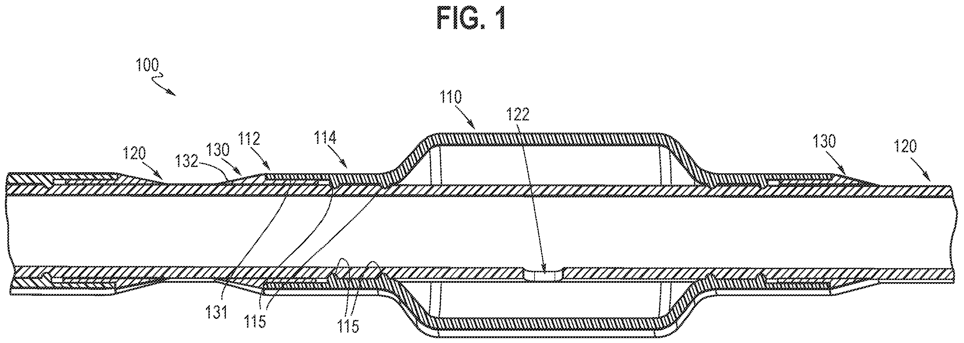

Figure 1 shows a cross-sectional view of an example inflatable device 100 including a balloon 110 attached to a shaft 120 via an overmold 130. In the example inflatable device 100, the balloon 110 encircles the shaft 120. In the example inflatable device 100, the outer face of the overmold 130 extends from an end 112 of the balloon 110 to a shut-off ridge 132.

In the example inflatable device 100, the shut-off ridge 132 includes a tapered shaping. In some cases, the shut-off ridge may cover only a part of the outer face of the overmold. In some cases, the shut-off ridge may cover the entire outer face of the overmold. The shut-off ridge 132 encircles the shaft 120 in an annular shape. The overmold 130 may meet the end of the balloon to form a flush joint. In other words, the outer surfaces of the balloon and overmold may be aligned to prevent and/or minimize discontinuity between the end of the balloon and the start of the outer surface of the taper. The tapered shape of the shut-off ridge 132 may be free of discontinuities (or other roughness such as flash) where the shut-off ridge 132 tapers into the shaft. As discussed above, discontinuities and/or other roughness/flash may cause tissue damage during use of the balloon and shaft device in a medical context.

The overmold 130 may include extension 131 under the balloon 110 via the inner surfaces of the overmold.

In the example inflatable device 100, the shaft includes an opening 122 that places the shaft in fluid (e.g., gas and/or liquid) contact or communication with the balloon. Accordingly, fluid may be sent through the shaft to increase the pressure inside the shaft 120. Via the opening 122, the pressure is transferred to the balloon, and the balloon 110 may inflate.

The shaft may be made from various materials such as polymeric materials, for example, silicone, polyvinyls, polyethlyenes, polyurethane, polyurethane co-polymers, polyvinyl chloride (PVC), and/or other polymers. Similarly, the balloon may be made out of polymeric materials, for example, silicone, silicone derivatives, or certain polyurethanes with high modulus of elasticity. In some cases, the balloon may be made out of polyethylene terephthalate (PET) or nylon. In some cases, the shaft may be more rigid than the balloon.

In a preferred embodiment, a silicone balloon is paired with a silicone shaft, in order to improve adhesion between the balloon and the shaft. Alternatively, in another preferred embodiment, a polyurethane balloon is paired with a shaft made of polyurethane, polyurethane co-polymers, or PVC, in order improve adhesion between the balloon and the shaft. In a preferred embodiment, the balloon is a conforming balloon, intended to expand or stretch in response to a volume of fluid injected into the balloon, to thereby fill the cross-section of a vessel or lumen in which the balloon is inserted, without causing tissue damage from high forces or pressure exerted on the walls of the vessel or lumen. In some preferred embodiments, the balloon may be pre-formed to have a defined shape when inflated. For example, a retrograde coronary sinus perfusion catheter may include a pre-formed bell-shaped balloon.

In some cases, the overmold may be formed using the material of the balloon and/or the shaft. Some materials, such as silicone and/or other self-adhering materials may form physically secure bonds when placed in contact with components made using that same material.

In the example inflatable device 100, the balloon includes two coupling ports 114 at the ends of the balloon 110. The shaft 120 is inserted through the two coupling ports and the overmolds 130 attach the balloon to the shaft. In some implementations, the balloon may have other numbers of coupling ports. For example, a balloon with a single coupling port may be affixed to the distal end of a shaft. A balloon with three coupling ports may be used at a shaft y-type junction. Caps (which may also be attached via overmolding) may be applied to coupling ports without an inserted shaft. Accordingly, a balloon with more coupling ports than used for shaft insertion may still be implemented.

The inner surface of the coupling ports 114 may be in contact with the outer surface of the shaft 120. In the example inflatable device 100, the inner surface of the coupling ports 114 may include ribs 115. The ribs 115 may encircle the shaft and may have an annular shape. The ribs 115 may increase contact pressure with the shaft surface 120. In some cases, the ribs 115 may increase blockage of material, for example, from escaping the balloon 110 via the coupling port 114.

As discussed above the example inflatable device 100 may be used in various medical contexts where an inflatable device with a conforming balloon is used to control fluid flow. For example, during a caesarian section procedure, the device may be inserted into the body an inflated to constrict blood flow in an artery, such as the uterine artery. The device may be used in other procedures to implement balloon occlusion, support of stent insertion, and/or other medical balloon inflation procedures.

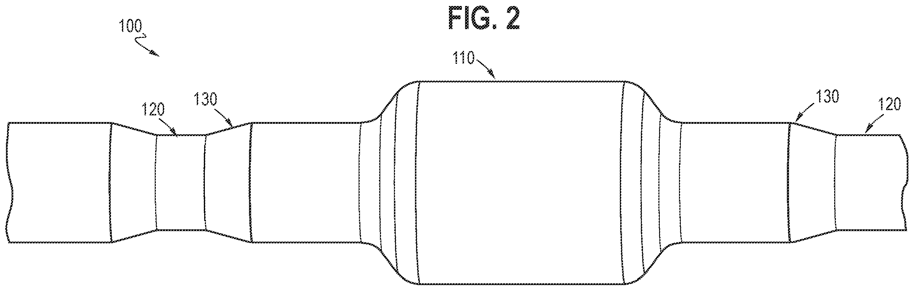

Figure 2 shows an alternate solid view of the example inflatable device 100 including the including the balloon attached to the shaft 120 via the overmold 130.

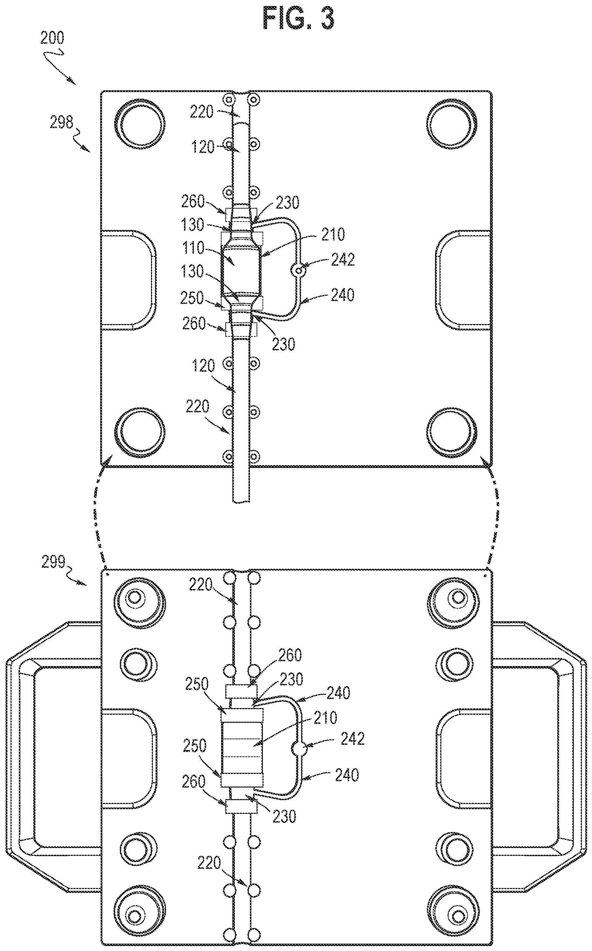

Figure 3 shows an example mold 200. The example mold 200 may include a shaft cavity 220 sized to accept a shaft 120.

The example mold 200 may include a balloon cavity 210 sized to accept a balloon 110 when an end of the shaft 120 is inserted into a coupling port 114 on the balloon 110. Accordingly, the mold may operate when a balloon and shaft are both inserted into their respective cavities 210, 220. The balloon cavity 210 may include a port wall that may be sized and shaped to secure the coupling port 114 of the balloon during molding.

The example mold 200 may include an overmolding cavity 230. The example mold 200 may include a material channel 240 configured to receive an overmolding medium. During operation of the mold, the material channel 240 may receive the material from an injection channel 242 and deliver the material (e.g., for formation of the overmold 130) to the overmolding cavity 230. The injection channel 242 may extend from the exterior of the mold to the material channel 240 to allow material to be supplied to the mold during molding.

The example mold 200 may include an inner shut-off 250 and an outer shut-off 260 spaced farther from the balloon than the inner shut-off 250. In some embodiments, the inner shut-off 250 and/or the outer shut-off 260 may comprise an annular structure that surrounds the shaft 120, or the shaft 120 and the balloon 110. The annular structure may comprise at least two pieces, for example, one piece being positioned in mold portion 298, and the other piece being position in mold portion 299, such that when mold portions 298 and 299 are closed together, the inner shut-off 250 and/or the outer shut-off 260 compress and seal around the shaft 120, or the shaft 120 and the balloon 110. In some embodiments, the inner shut-off 250 and/or the outer shut-off 260 may be constructed of a heat resistance polyamide, such as, for example, TORLON ^{®}.

The overmolding cavity 230 may be situated between the inner 250 and outer shut-offs 260. During operation of the mold and when material is supplied to the overmolding cavity 230, the inner 250 and outer shut-offs 260 may work to shape the end of the overmold. As an illustration, for the example inflatable device 100, the outer shut-off 260 (in concert with the walls of the overmolding cavity 230) shapes the resulting overmold to create a taper-shaped shut-off ridge (e.g., without flash or other roughness). In the example inflatable device 100, the inner shut-off 250 may be positioned and sized to create a flush joint between the overmold and the outer surface of the coupling port 114 of the balloon 110. The flush joint may be ensured by setting the inner surface of the inner shut-off 250 to be flush with the port wall of the balloon cavity 210. In the example mold 200, the inner shut-off 250 may secure the coupling port 114 of the balloon 110 and perform the function of the port wall. In some cases, using a single part for both the port wall and inner shut-off 250 may help ensure a flush joint for the overmold 130.

When material is injected into the mold during molding, the overmolding cavity, the material channel, and/or the injection channel may be filled (or partially filled) with the molding material. In some cases, the molding material may include the material of the balloon and/or shaft. In some cases, the material may be silicone. After the material is injected into the mold, the material may be allowed to cure and/or a curing processing may be affirmatively applied. For example, for silicone, the mold may be raised to a reaction temperature at which silicone will cure after exposure. In some cases, the reaction temperature may include a melting temperature for a material or other temperature at which one or more physical properties of the material may change.

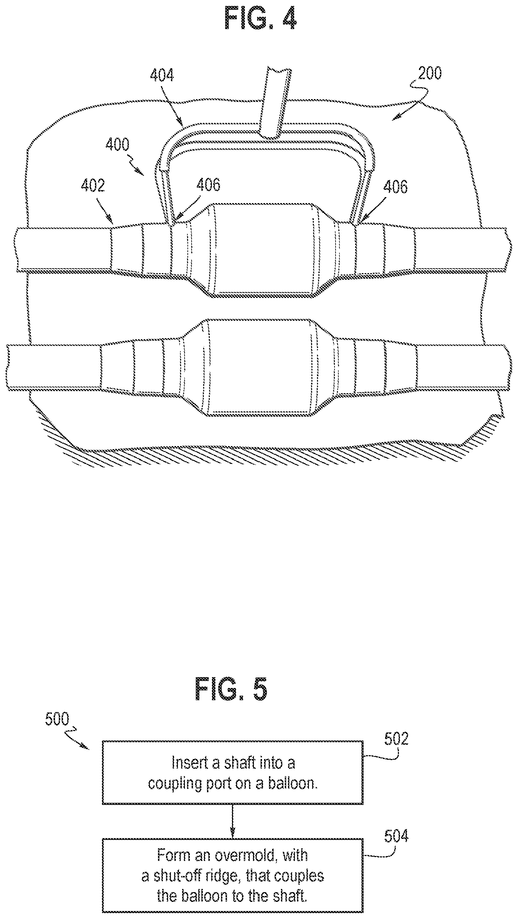

Referring briefly to Figure 4, an example after-molding device 400 is shown. After molding, the molded inflatable device 402 may be removed from the mold 200. In some cases, because the injection and/or material channels may be at least partially filled, a channel form 404 may form attached to the molded inflatable device 402. The channel form 404 may have one or more attachment points at the overmolds 406 on the inflatable device 402. The channel form 404 may be removed from the inflatable device 402, for example, by cutting, ablation, pulling, or other removal. In some cases, the removal may result in a smooth surface with continuity with the surrounding surface of the overmold 406. In some cases, the removal may result in a cut-point (or other removal technique) artifact on the surface of the overmold 406 at the location of the removal of the channel form. The artifact may include a visible discontinuity, a surface quality discontinuity, and/or other discontinuity with the surrounding overmold surface.

Referring again to Figure 3, the example mold 200 may include multiple complementary parts that may be opened and/or disassembled to allow for loading of the mold (e.g., with the balloon and the shaft) prior to molding. In the example mold 200, the mold may include two complementary portions 298, 299 that may close together to hold the balloon and shaft relative to one another and the shut-offs during molding. In some implementations, the shut-offs may be replaceable to allow modification of the shape/size of the shut-off ridge and/or the shape of the end of the overmold 130 abutting the coupling port 114 of the balloon 110.

Figure 5 shows an example method 500 for fabricating an inflatable device. The device may be fabricated by inserting a shaft into a coupling port on a balloon (502). Then, forming an overmold, with a shut-off ridge, that couples the balloon to the shaft (504) works to join the two components. The example method 500 may be expanded and/or altered to support fabrication of an inflatable device with any of or any combination of the features discussed above. Further, the example method 500 may performed using the example mold 200 (or other appropriately designed mold). In some cases, a resultant product may be specified as being a result of implementing the example method 500.

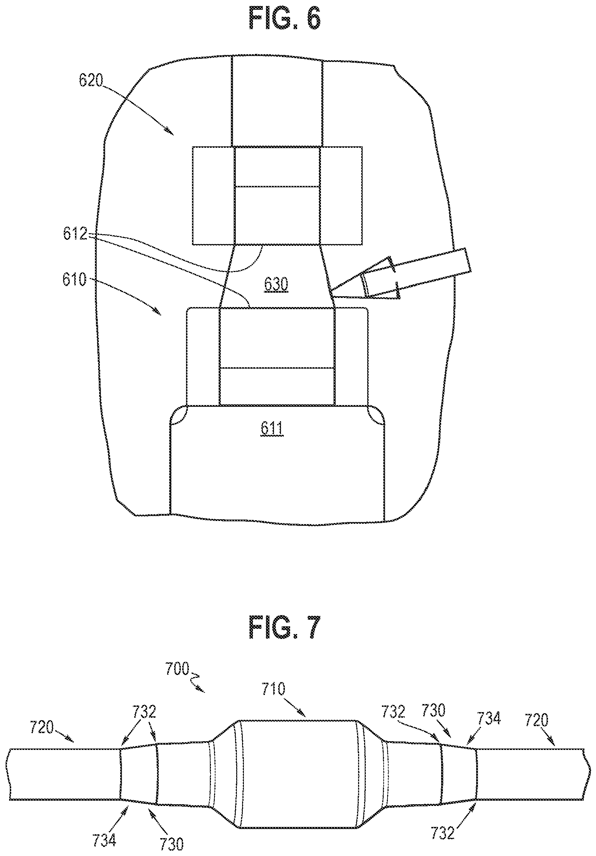

Figure 6 shows a detail view of example shut-offs 610, 620. The detail view shows internal structure of the example shut-offs 610, 620. The example inner shut-off 610 is positioned/sized to create a flush joint 612 where the balloon 611 abuts the overmold 630. The outer shut-off 620 is positioned/shaped to create a shut-off ridge flush with the shaft at the end of the overmold 630 away from the balloon 611. Other overmold shapes are possible.

Figure 7 shows an example of a completed inflation device 700 that includes a balloon 710, a shaft 720, and overmolds 730. The example inflation device 700 has flush joints 732 created by an inner shut off and tapered shut-off ridges 734 shaped by an outer shut-off.

Various implementations have been specifically described. However, many other implementations are also possible.

## Claims

1. A device (100) including:
a catheter shaft (120);
a balloon (110) in pressure communication with the catheter shaft (120); and
a first overmold (130) coupling the balloon (110) to the catheter shaft, the first overmold (130) extending from the balloon (110) to a shut-off ridge (132).

2. The device of any of the preceding claims, where the shut-off ridge forms an annular shape around the catheter shaft (120).

3. The device of any of the preceding claims, where the first overmold (130) is formed using a material identical to a material of the balloon (110), where optionally, the material is silicone.

4. The device of any one of claims 1 to 3, where the balloon (110) includes a first coupling port (114) that accepts the catheter shaft (120).

5. The device of claim 4, where:
the first overmold (130) is flush to an outside of the first coupling port (114) on the balloon (110); and
the first overmold (130) includes an extension into the first coupling port (114) between the balloon and the first catheter shaft inside the first coupling port.

6. The device of claim 4, where:
the first coupling port (114) includes an internal rib (115), the internal rib in contact with an outside of the catheter shaft, where:
optionally, the internal rib has an annular shape; and
optionally, coupling port includes multiple internal ribs in contact with the outside of the catheter shaft (120).

7. The device of claim 4, where:
the balloon (110) includes a second coupling port (114) opposite the first coupling port;
the catheter shaft (120) extends through the balloon (110) through the first and second coupling ports, and
optionally wherein the device further includes a second overmold (130) coupling the balloon to the catheter shaft at the second coupling port (114).

8. The device of any of the preceding claims, where the first overmold (130) tapers between the balloon (110) and the shut-off ridge (132).

9. The device of any of the preceding claims, where the catheter shaft includes a wall made from a material more rigid than that of the balloon (110), where:
optionally, the material is a polymeric material; and
optionally, the material is silicone.

10. The device of any of the preceding claims, where the first overmold (130) includes a cut-point artifact where an injection form was removed after molding.

11. The device of any of the preceding claims, where either:
the first overmold (130) is formed from the material of the catheter shaft (120); or
the shut-off ridge (132) has a flash-free shaping.

12. The device of any of the preceding claims, where the device is configured to perform:
uterine artery balloon occlusion;
endovascular balloon occlusion;
any other balloon occlusion;
an insertion of a stent; and/or
as any other inflatable medical device.

13. A device comprising:
a mold block (200) including:
a shaft channel (220) sized to accept a catheter shaft (120);
a balloon cavity (210) sized to accept a balloon (110) when an end of the catheter shaft (120) is inserted into a coupling port (114) on the balloon (110);
a material channel (240) configured to receive an overmolding medium;
an inner shut-off (250);
an outer shut-off (260) spaced father from the balloon cavity (210) than the inner shut-off; and
an overmolding cavity (230) between the inner and outer shut-offs, the material channel extending into the overmolding cavity.

14. The device of claim 13, where either:
the mold block includes two complementary portions (298, 299) that couple to one another during molding; or
the mold is configured to operate above a reaction temperature to cure silicone.

15. The device of claim 13 or 14, where the mold block further includes an injection channel (242) extending from an outside surface of the mold to the material channel (240).

16. The device of any of claims 13-15, where either:
the inner shut-off is set flush to a port wall of the balloon cavity, the port wall configured to secure the coupling port of the balloon (110), when the balloon is inserted into the balloon cavity; or
an inner face of the inner shut-off is shaped to form a shut-off ridge that surrounds the catheter shaft (120) during molding.

## Patentansprüche

1. Vorrichtung (100), Folgendes umfassend:
einen Katheterschaft (120);
einen Ballon (110) in Druckverbindung mit dem Katheterschaft (120); und
einen ersten Überguss (130), der den Ballon (110) mit dem Katheterschaft verbindet, wobei sich der erste Überguss (130) von dem Ballon (110) zu einem Verschlusskamm (132) erstreckt.

2. Vorrichtung nach einem der vorhergehenden Ansprüche, wobei der Verschlusskamm eine ringförmige Gestalt um den Katheterschaft (120) herum ausbildet.

3. Vorrichtung nach einem der vorhergehenden Ansprüche, wobei der erste Überguss (130) unter Verwendung eines Materials ausgebildet ist, das mit einem Material des Ballons (110) identisch ist, wobei das Material gegebenenfalls Silikon ist.

4. Vorrichtung nach einem der Ansprüche 1 bis 3, wobei der Ballon (110) einen ersten Verbindungsport (114) umfasst, der den Katheterschaft (120) aufnimmt.

5. Vorrichtung nach Anspruch 4, wobei:
der erste Überguss (130) bündig mit einer Außenseite des ersten Verbindungsports (114) auf dem Ballon (110) ist; und
der erste Überguss (130) eine Verlängerung in den ersten Verbindungsport (114) zwischen dem Ballon und dem ersten Katheterschaft innerhalb des ersten Verbindungsports umfasst.

6. Vorrichtung nach Anspruch 4, wobei:
der erste Verbindungsport (114) eine innere Rippe (115) umfasst, wobei die innere Rippe mit einer Außenseite des Katheterschafts in Kontakt steht, wobei:
die innere Rippe gegebenenfalls eine ringförmige Gestalt aufweist; und
der Verbindungsport gegebenenfalls mehrere innere Rippen in Kontakt mit der Außenseite des Katheterschafts (120) umfasst.

7. Vorrichtung nach Anspruch 4, wobei:
der Ballon (110) einen zweiten Verbindungsport (114) gegenüber dem ersten Verbindungsport umfasst;
der Katheterschaft (120) sich durch den Ballon (110) durch den ersten und durch den zweiten Verbindungsport erstreckt, und
wobei die Vorrichtung gegebenenfalls weiterhin einen zweiten Überguss (130) umfasst, der den Ballon an dem zweiten Verbindungsport (114) mit dem Katheterschaft verbindet.

8. Vorrichtung nach einem der vorhergehenden Ansprüche, wobei sich der erste Überguss (130) zwischen dem Ballon (110) und dem Verschlusskamm (132) verjüngt.

9. Vorrichtung nach einem der vorhergehenden Ansprüche, wobei der Katheterschaft eine Wand umfasst, die aus einem Material hergestellt ist, das steifer ist als dasjenige des Ballons (110), wobei:
das Material gegebenenfalls ein Polymermaterial ist; und
das Material gegebenenfalls Silikon ist.

10. Vorrichtung nach einem der vorhergehenden Ansprüche, wobei der erste Überguss (130) ein Verschlusspunkt-Artefakt umfasst, wo eine Spritzgussform nach einem Gießen entfernt wurde.

11. Vorrichtung nach einem der vorhergehenden Ansprüche, wobei entweder:
der erste Überguss (130) aus dem Material des Katheterschafts (120) ausgebildet ist; oder
der Verschlusskamm (132) eine gratfreie Gestaltung aufweist.

12. Vorrichtung nach einem der vorhergehenden Ansprüche, wobei die Vorrichtung eingerichtet ist, um Folgendes durchzuführen:
einen Ballonverschluss der Gebärmutterarterie;
einen endovaskulären Ballonverschluss;
jeden anderen Ballonverschluss;
eine Einführung eines Stents; und/oder
wie jede andere aufblasbare medizinische Vorrichtung.

13. Vorrichtung, Folgendes umfassend:
einen Gussblock (200), Folgendes umfassend:
einen Schaftkanal (220), der bemessen ist, dass er einen Katheterschaft (120) aufnimmt;
einen Ballonhohlraum (210), der bemessen ist, um einen Ballon (110) aufzunehmen, wenn ein Ende des Katheterschafts (120) in einen Verbindungsport (114) an dem Ballon (110) eingeführt wird;
einen Materialkanal (240), der eingerichtet ist, um ein Übergussmedium aufzunehmen;
einen inneren Verschluss (250);
einen äußeren Verschluss (260), der von dem Ballonhohlraum (210) weiter beabstandet ist, als der innere Verschluss; und
einen Übergusshohlraum (230) zwischen dem inneren und dem äußeren Verschluss, wobei sich der Materialkanal in den Übergusshohlraum erstreckt.

14. Vorrichtung nach Anspruch 13, wobei entweder:
der Gussblock zwei komplementäre Abschnitte (298, 299) umfasst, die während des Gießens miteinander verbunden sind; oder
die Gussform eingerichtet ist, um über einer Reaktionstemperatur zum Härten von Silikon zu arbeiten.

15. Vorrichtung nach Anspruch 13 oder 14, wobei der Gussblock weiterhin einen Einspritzkanal (242) umfasst, der sich von einer Außenfläche der Gussform zu dem Materialkanal (240) erstreckt.

16. Vorrichtung nach einem der Ansprüche 13 bis 15, wobei entweder:
der innere Verschluss bündig auf eine Portwand des Ballonhohlraums eingestellt ist, wobei die Portwand eingerichtet ist, um den Verbindungsport des Ballons (110) zu sichern, wenn der Ballon in den Ballonhohlraum eingeführt ist; oder
eine Innenfläche des inneren Verschlusses geformt ist, um einen Verschlusskamm auszubilden, der den Katheterschaft (120) beim Gießen umgibt.

## Revendications

1. Dispositif (100) comprenant :
un arbre de cathéter (120) ;
un ballonnet (110) en communication de pression avec l'arbre de cathéter (120) ; et
un premier surmoulage (130) couplant le ballonnet (110) à l'arbre de cathéter, le premier surmoulage (130) s'étendant du ballonnet (110) à une nervure d'arrêt (132).

2. Dispositif selon l'une quelconque des revendications précédentes, la nervure d'arrêt formant une forme annulaire autour de l'arbre de cathéter (120).

3. Dispositif selon l'une quelconque des revendications précédentes, le premier surmoulage (130) étant formé à l'aide d'un matériau identique à un matériau du ballonnet (110), éventuellement, le matériau étant du silicone.

4. Dispositif selon l'une quelconque des revendications 1 à 3, le ballonnet (110) comprenant un premier orifice de couplage (114) qui reçoit l'arbre de cathéter (120).

5. Dispositif selon la revendication 4,
le premier surmoulage (130) affleurant à l'extérieur du premier orifice de couplage (114) sur le ballonnet (110) ; et
le premier surmoulage (130) comprenant une extension dans le premier orifice de couplage (114) entre le ballonnet et le premier arbre de cathéter à l'intérieur du premier orifice de couplage.

6. Dispositif selon la revendication 4,
le premier orifice de couplage (114) comprenant une nervure interne (115), la nervure interne étant en contact avec l'extérieur de l'arbre de cathéter,
éventuellement, la nervure interne ayant une forme annulaire ; et
éventuellement, l'orifice de couplage comprenant de multiples nervures internes en contact avec l'extérieur de l'arbre de cathéter (120).

7. Dispositif selon la revendication 4,
le ballonnet (110) comprenant un second orifice de couplage (114) opposé au premier orifice de couplage ;
l'arbre de cathéter (120) s'étendant à travers le ballonnet (110) en passant par le premier et le second orifice de couplage, et
éventuellement, le dispositif comprenant en outre un second surmoulage (130) couplant le ballonnet à l'arbre de cathéter au niveau du second orifice de couplage (114).

8. Dispositif selon l'une quelconque des revendications précédentes, le premier surmoulage (130) se rétrécissant entre le ballonnet (110) et la nervure d'arrêt (132).

9. Dispositif selon l'une quelconque des revendications précédentes, l'arbre de cathéter comprenant une paroi faite d'un matériau plus rigide que celui du ballonnet (110),
éventuellement, le matériau étant un matériau polymère ; et
éventuellement, le matériau étant du silicone.

10. Dispositif selon l'une quelconque des revendications précédentes, le premier surmoulage (130) comprenant un artefact de point de coupe où une forme d'injection a été retirée après le moulage.

11. Dispositif selon l'une quelconque des revendications précédentes,
le premier surmoulage (130) étant formé à partir du matériau de l'arbre de cathéter (120) ; ou
la nervure d'arrêt (132) ayant une forme sans bavure.

12. Dispositif selon l'une quelconque des revendications précédentes, le dispositif étant configuré pour réaliser :
une occlusion par ballonnet de l'artère utérine ;
une occlusion par ballonnet endovasculaire ;
toute autre occlusion par ballonnet ;
l'insertion d'une endoprothèse ; et/ou
tout autre dispositif médical gonflable.

13. Dispositif comprenant :
un bloc de moulage (200) comprenant :
un canal d'arbre (220) dimensionné pour recevoir un arbre de cathéter (120) ;
une cavité pour ballonnet (210) dimensionnée pour recevoir un ballonnet (110) lorsqu'une extrémité de l'arbre de cathéter (120) est insérée dans un orifice de couplage (114) sur le ballonnet (110) ;
un canal de matériau (240) configuré pour recevoir un milieu de surmoulage ;
un obturateur interne (250) ;
un obturateur externe (260) plus éloigné de la cavité de ballonnet (210) que l'obturateur interne ; et
une cavité de surmoulage (230) entre les obturateurs interne et externe, le canal de matériau s'étendant dans la cavité de surmoulage.

14. Dispositif selon la revendication 13,
le bloc de moulage comprenant deux parties complémentaires (298, 299) qui se couplent l'une à l'autre pendant le moulage ; ou
le moule étant configuré pour fonctionner au-dessus d'une température de réaction pour durcir le silicone.

15. Dispositif selon la revendication 13 ou 14, le bloc de moule comprenant en outre un canal d'injection (242) s'étendant d'une surface extérieure du moule au canal de matériau (240).

16. Dispositif selon l'une quelconque des revendications 13 à 15,
l'obturateur interne étant placé au niveau d'une paroi d'orifice de la cavité de ballonnet, la paroi d'orifice étant configurée pour fixer l'orifice de couplage du ballonnet (110), lorsque le ballonnet est inséré dans la cavité de ballonnet ; ou
une face interne de l'obturateur interne étant façonnée pour former une nervure d'arrêt qui entoure l'arbre de cathéter (120) pendant le moulage.
